# EUROPEAN PATENT APPLICATION

(11) **EP 4 535 219 A1**
(43) Date of publication of application: **09.04.2025**
(21) Application number: 23815520.4
(22) Date of filing: 16.03.2023
(51) Int. Cl.: G06F 30/27, C04B 38/00, G06F 30/10

(54) **POROUS BODY DESIGN METHOD AND POROUS BODY MANUFACTURING METHOD**

(30) Priority: 31.05.2022 JP 2022089023
(71) Applicant: NGK Insulators, Ltd., Nagoya city, Aichi 467-8530 (JP); Institute of Science Tokyo, Tokyo 152-8550 (JP)
(72) Inventor: SOKAWA, Shingo, Nagoya-city, Aichi 467-8530 (JP); HASHIMOTO OKA Yuki, Nagoya-city, Aichi 467-8530 (JP); TAKAHASHI, Tomonori, Nagoya-city, Aichi 467-8530 (JP); OKAWARA, Shinichi, Tokyo 152-8550 (JP); YASUDA, Tomoki, Tokyo 152-8550 (JP); YOSHIKAWA, Shiro, Tokyo 152-8550 (JP); MATSUMOTO, Hideyuki, Tokyo 152-8550 (JP)
(74) Representative: Mewburn Ellis LLP
(86) International application number: PCT/JP2023/010482
(87) International publication number: WO 2023/233758

(57) **Abstract**

A porous body design method using a computer causes the computer to execute, a plurality of times, a structure generation process of virtually generating a three-dimensional structure of a porous body on the computer on the basis of generation parameter values for generating the porous body, a characteristic prediction/calculation process of predicting or calculating the characteristics of the porous body having the three-dimensional structure generated by the structure generation process, an evaluation process of evaluating the characteristics of the porous body predicted or calculated by the characteristic prediction/calculation process, and an optimization process of changing the generation parameter values to search for optimal generation parameter values, in which the three-dimensional structure of the porous body is determined on the basis of evaluation results of the characteristics of the porous body obtained by the evaluation process.

## Description

### [Technical Field]

The present invention relates to a porous body design method and a porous body manufacturing method.

### [Background Art]

In the related art, porous bodies in which many pores are disposed three-dimensionally in a material have been widely used for various applications, such as purification of exhaust gases emitted from internal combustion engines such as gasoline and diesel engines. In designing such porous bodies, it is necessary to determine a three-dimensional structure of a porous body so that appropriate characteristics can be obtained in accordance with the application.

Porous body design methods of the related art include, for example, a method of creating several prototypes of porous bodies on the basis of a designer's experience and intuition, measuring a three-dimensional structure and characteristics of each of these prototypes, and then determining a final three-dimensional structure of the porous body after ascertaining a relationship between the three-dimensional structure and the characteristics of the porous body. However, with such a design method, it takes a lot of time and laborious effort to create prototypes and measure the characteristics, and thus there is a limitation on a range in which a relationship between a three-dimensional structure and characteristics can be searched for. For this reason, there is a problem in that it is not necessarily possible to determine a three-dimensional structure of a porous body from which desired characteristics can be obtained.

On the other hand, in recent years, a method using simulation software has also become known, for example, as disclosed in PTL 1. With such simulation software, a three-dimensional model of a porous body can be created, and characteristics of the porous body can be obtained from this three-dimensional model by calculation.

### [Citation List]

### [Patent Literature]

[PTL 1] Japanese Patent No. 6940786

### [Summary of Invention]

### [Technical Problem]

In the method disclosed in PTL 1, a sample is created from a partition piece cut out from an exhaust gas purification filter, and continuous cross-sectional images, which are obtained by imaging the sample with an X-ray CT scanner, are read by simulation software to form a three-dimensional model, thereby deriving the number of continuous holes of the exhaust gas purification filter, i.e., a porous body. Thus, it is necessary to create the exhaust gas purification filter in advance, and there is still a problem in that a lot of time and laborious effort are required for prototyping.

In view of the above-mentioned problems, an object of the present invention is to provide a useful technology that makes it possible to determine an optimal structure early in the design of a porous body.

### [Solution to Problem]

A porous body design method according to the present invention is a design method of porous body using a computer and causes the computer to execute, a plurality of times, a structure generation process of virtually generating a three-dimensional structure of a porous body on the computer on the basis of generation parameter values for generating the porous body, a characteristic prediction/calculation process of predicting or calculating the characteristics of the porous body having the three-dimensional structure generated by the structure generation process, an evaluation process of evaluating the characteristics of the porous body predicted or calculated by the characteristic prediction/calculation process, and an optimization process of changing the generation parameter values to search for optimal generation parameter values, wherein the three-dimensional structure of the porous body is determined on the basis of evaluation results of the characteristics of the porous body obtained by the evaluation process.

A porous body manufacturing method according to the present invention is a method of manufacturing the porous body using three-dimensional shape data based on the three-dimensional structure of the porous body determined by the porous body design method.

### [Advantageous Effects of Invention]

According to the present invention, it is possible to provide a useful technology that makes it possible to determine an optimal structure early in the design of a porous body.

### [Brief Description of Drawings]

[Fig. 1]

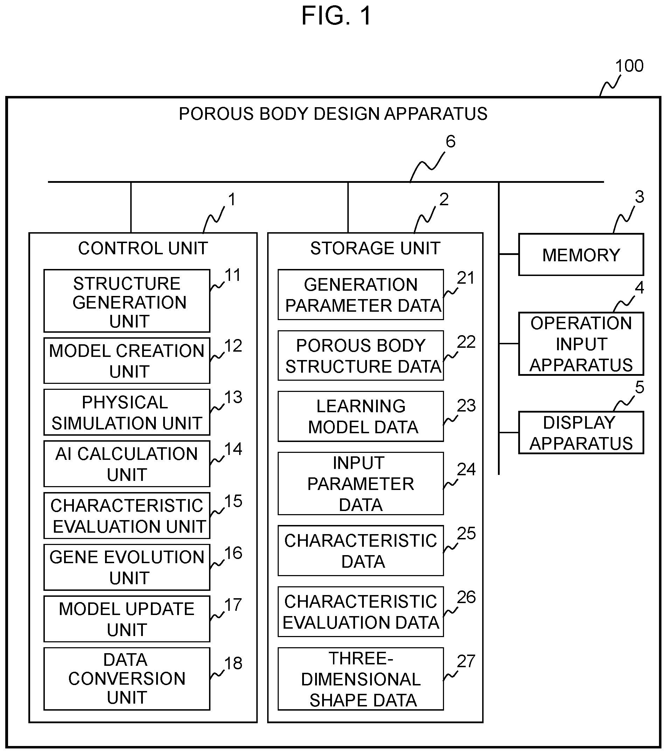

   Fig. 1 is a block diagram showing a configuration of a porous body design apparatus according to an embodiment of the present invention.
[Fig. 2]

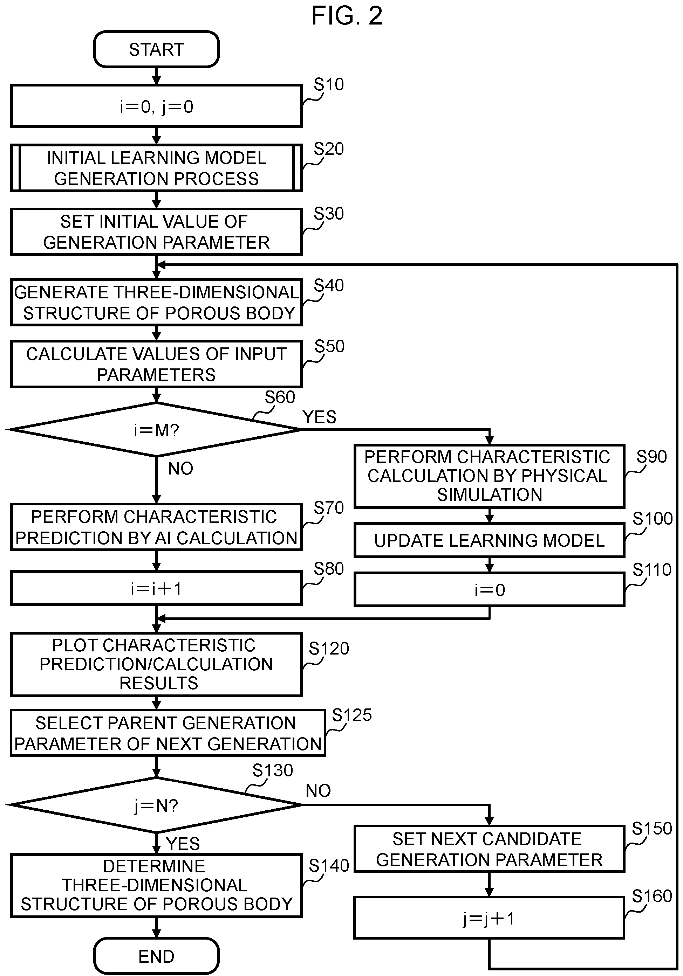

   Fig. 2 is a flowchart showing a flow of processing of a porous body design apparatus according to an embodiment of the present invention.
[Fig. 3]

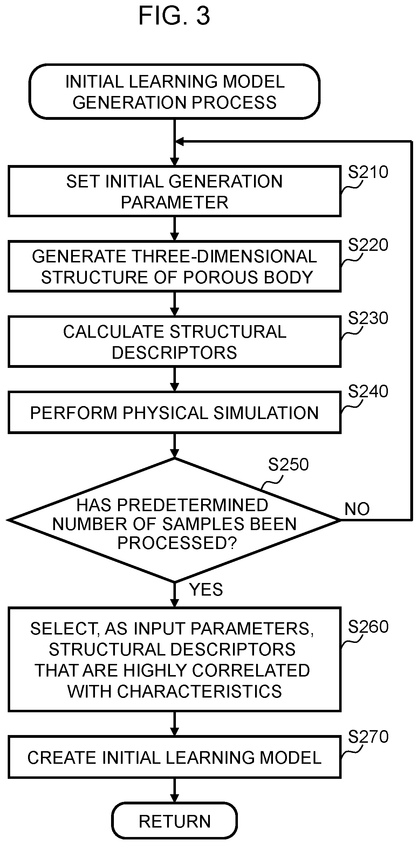

   Fig. 3 is a flowchart showing details of an initial learning model generation process.
[Fig. 4]

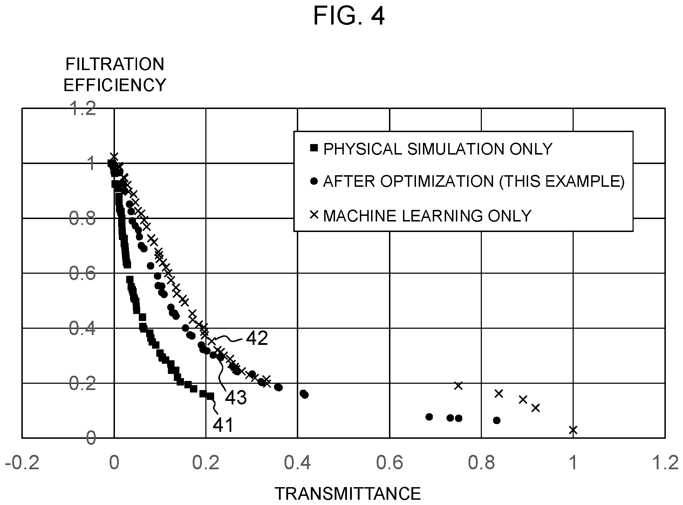

   Fig. 4 is a diagram showing comparison between a three-dimensional structure of a porous body obtained by the porous body design apparatus of the present embodiment and a three-dimensional structure of a porous body according to a comparative example.
[Fig. 5]

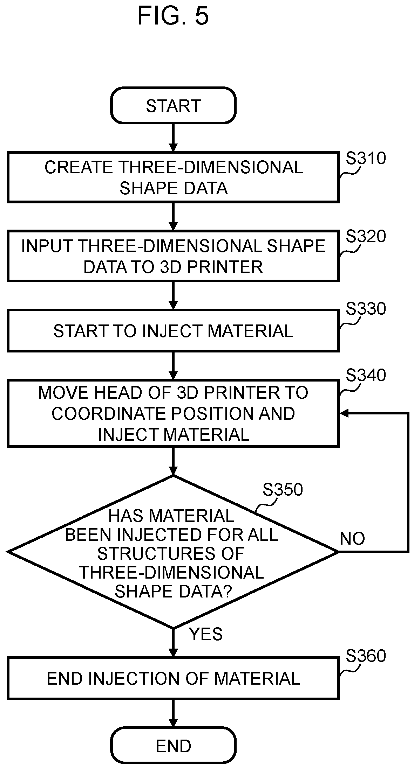

   Fig. 5 is a flowchart showing a flow of a porous body manufacturing process using a 3D printer.

### [Description of Embodiments]

Hereinafter, embodiments of the present invention will be described with reference to the drawings. In the following embodiment, description will be given of a porous body design apparatus that can determine a three-dimensional structure of a porous body having desired characteristics in a relatively short period of time by being operated by a designer who designs the porous body.

Fig. 1 is a block diagram showing a configuration of a porous body design apparatus according to an embodiment of the present invention. A porous body design apparatus 100 shown in Fig. 1 is a computer that includes a control unit 1, a storage unit 2, a memory 3, an operation input apparatus 4, and a display apparatus 5, and is configured by connecting these apparatuses to each other via a bus 6.

The control unit 1 is configured using, for example, a central processing unit (CPU) and performs various processes and calculations for operating the porous body design apparatus 100. The control unit 1 executes programs stored in the storage unit 2 to realize functional blocks of a structure generation unit 11, a model creation unit 12, a physical simulation unit 13, an AI calculation unit 14, a characteristic evaluation unit 15, a gene evolution unit 16, a model update unit 17, and a data conversion unit 18. Details of these functional blocks will be described later. Some or all of the functions of the control unit 1 may be realized using devices other than the CPU, such as a graphics processing unit (GPU), a field programmable gate array (FPGA), and an application specific integrated circuit (ASIC).

The storage unit 2 is configured using a magnetic storage apparatus such as a hard disk drive (HDD) or a large-capacity non-volatile memory apparatus such as a solid state drive (SSD), and stores programs executed by the control unit 1 and various types of information used in the processing of the control unit 1. The information stored in the storage unit 2 includes data including generation parameter data 21, porous body structure data 22, learning model data 23, input parameter data 24, characteristic data 25, characteristic evaluation data 26, and three-dimensional shape data 27. Details of the data will be described later.

The memory 3 is configured using a high-speed volatile storage apparatus such as a dynamic random access memory (DRAM), and is used as a working area when the control unit 1 executes a program.

The operation input apparatus 4 is an apparatus for detecting a user's operation input and is configured using, for example, a keyboard or a mouse. The display apparatus 5 is an apparatus that displays processing results of the porous body design apparatus 100 on a screen and presents them to the user, and is configured using, for example, a liquid crystal display or an organic EL display. Another computer or smartphone that can communicate with the porous body design apparatus 100 may be used as the operation input apparatus 4 or the display apparatus 5.

The configuration of the porous body design apparatus 100 shown in Fig. 1 may be physically constructed on one computer or may be constructed to be distributed on a plurality of computers. The porous body design apparatus 100 may also be realized by a cloud computer installed on a cloud, a virtual machine operating in a virtual environment, or the like.

Next, the functional blocks of the control unit 1, that is, the structure generation unit 11, the model creation unit 12, the physical simulation unit 13, the AI calculation unit 14, the characteristic evaluation unit 15, the gene evolution unit 16, the model update unit 17, and the data conversion unit 18, will be described. In the porous body design apparatus 100, the control unit 1 operates as these functional blocks, making it possible to determine a three-dimensional structure of a porous body having desired characteristics.

The structure generation unit 11 sets the values of generation parameters for generating a porous body to be evaluated. The generation parameters are physical quantities that can be adjusted in a manufacturing process when the porous body is actually manufactured, and include, for example, an average and standard deviation of particle diameters of spheres and ellipsoids contained in a base material that is the raw material of the porous body, a volume ratio of the spheres to the ellipsoids in the base material, the amount and particle diameter of a pore-forming material that is mixed with the base material to form voids in the porous body, the degree of sintering of the base material, and the like. The structure generation unit 11 can set the values of these generation parameters on the basis of, for example, a user's operation contents input via the operation input apparatus 4**.** The values of the generation parameters set by the structure generation unit 11 are stored in the storage unit 2 as generation parameter data 21.

Furthermore, the structure generation unit 11 performs a structure generation process for virtually generating a three-dimensional structure of the porous body to be evaluated on the porous body design apparatus 100 on the basis of the values of the generation parameters that are set as described above. For this process, for example, GeoDict, a microstructure simulation software developed by Math2Market GmbH, can be used. Data representing the three-dimensional structure of the porous body generated by the structure generation unit 11 performing the structure generation process is stored in the storage unit 2 as porous body structure data 22.

The model creation unit 12 creates a learning model capable of predicting characteristics corresponding to the three-dimensional structure of the porous body generated by the structure generation unit 11. The learning model is a prediction model that learns a relationship between the three-dimensional structure of the porous body and the characteristics, and is also referred to as a surrogate model. For example, by using a support vector machine (SVM), it is possible to generate a support vector regression that makes it possible to predict the characteristics of the porous body from feature data of the three-dimensional structure of the porous body as a learning model. A method of constructing a learning model using an SVM is known, and is described, for example, by Tomoki Yasuda, Shinichi Ookawara, Shiro Yoshikawa, and Hideyuki Matsumoto in "Machine learning and data-driven characterization framework for porous materials: Permeability prediction and channeling defect detection", Chemical Engineering Journal 420 (2021) 130069 DOI: 10.1016/j.cej.2021.130069. Information on the learning model created by the model creation unit 12 is stored in the storage unit 2 as learning model data 23. A learning model created in the past by the model creation unit 12 for a different porous body, rather than the porous body generated by the structure generation unit 11, may be used as learning model data 23. Alternatively, a learning model acquired from the outside may be stored in the storage unit 2 as the learning model data 23.

Before starting to create a learning model in the model creation unit 12, a predetermined number of samples of the three-dimensional structure of the porous body are generated in advance through the structure generation process of the structure generation unit 11. Then, various structural features of each sample are represented by the values of a plurality of types of predetermined structural descriptors. Structural descriptors that represent a three-dimensional structure of a porous body include, for example, a porosity, a solid fraction, a specific surface area, a pore diameter, a particle diameter, structural uniformity of pores or solid, a pore cord length, and a solid cord length. More specifically, the structural uniformity of the pores or solid means the variance of values related to the pores or solid calculated for each area by dividing the porous body into a plurality of areas. Examples of the values related to the pores or solid calculated for each area include the above-mentioned porosity, solid fraction, specific surface area, pore diameter, particle diameter, and the like. The pore cord length is the continuous length of pores in a predetermined direction in the porous body, and the solid cord length is the continuous length of the solid in a predetermined direction in the porous body. The model creation unit 12 selects, from among these structural descriptors, a plurality of structural descriptors that contribute highly to the characteristics of the porous body as input parameters for the learning model. Then, a learning model that predicts characteristics from the input parameters is created using the value of each input parameter of each sample and the characteristics of the porous body obtained for each sample by the following physical simulation performed by the physical simulation unit 13.

The physical simulation unit 13 performs physical calculations based on the three-dimensional structure of the porous body generated by the structure generation unit 11, and performs a physical simulation when a predetermined material is passed through the porous body. Thereby, for example, when a fluid such as exhaust gas from an internal combustion engine is passed through the porous body, values such as the permeability of the fluid and filtration efficiency (capture efficiency) of particulate matter (PM) contained in the fluid can be calculated as the characteristics of the porous body. In addition, the characteristics of the porous body to be calculated may include mechanical strength characteristics, electrochemical characteristics, thermal conductivity characteristics, heat exchange characteristics, electrical conductivity characteristics, gas adsorption characteristics, gas purification performance, catalyst coating properties, and removal efficiency of substances captured in the porous body. Results of the calculation of the characteristics of the porous body by the physical simulation unit 13 are stored in the storage unit 2 as the characteristic data 25. The physical simulation unit 13 can be realized using, for example, the functions of the above-mentioned GeoDict.

The AI calculation unit 14 performs calculation related to characteristic prediction of the porous body by artificial intelligence (AI) by using the learning model created by the model creation unit 12. The AI calculation unit 14 inputs the values of the above-mentioned input parameters obtained for the three-dimensional structure of the porous body into a learning model, thereby performing AI calculations using a well-known AI method such as support vector regression, and can predict the characteristics of the porous body. The values of the input parameters used in the AI calculation unit 14 and the obtained characteristic prediction results for the porous body are stored in the storage unit 2 as the input parameter data 24 and the characteristic data 25, respectively.

The characteristic evaluation unit 15 performs evaluation processing for evaluating the characteristics of the porous body calculated or predicted by the physical simulation unit 13 or the AI calculation unit 14. The characteristic evaluation unit 15 performs evaluation processing for the characteristics of the porous body, for example, by plotting each calculated or predicted characteristic value on a graph. Other methods may be used as long as the characteristics of the porous body can be appropriately evaluated. For example, an evaluation score for the characteristics of the porous body may be calculated from each characteristic value, or it may be determined whether each characteristic value satisfies a predetermined standard value and an OK/NG determination may be made for the characteristics of the porous body on the basis of the determination result. The characteristic evaluation results for the porous body which are obtained by the characteristic evaluation unit 15 are stored in the storage unit 2 as the characteristic evaluation data 26**.**

The gene evolution unit 16 performs a process of changing the values of the generation parameters. The gene evolution unit 16 performs a gene evolution process using a well-known calculation method such as a genetic algorithm, thereby changing the value of each generation parameter represented by the generation parameter data 21 to improve the characteristics of the porous body generated by the parameter value. By repeating such a gene evolution process by the gene evolution unit 16, it is possible to successively update the contents of the generation parameter data 21 and search for optimal parameter values.

The model update unit 17 performs an update process for the learning model on the basis of the results of the physical simulation performed by the physical simulation unit 13. As will be described below, the physical simulation unit 13 performs a physical simulation based on the three-dimensional structure of the porous body each time the AI calculation unit 14 performs an AI calculation a predetermined number of times. When the physical simulation unit 13 performs a physical simulation in this manner, the model update unit 17 performs an update process for the learning model by using the results and updates the content of the learning model data 23. Thereby, the results of the physical simulation are reflected in the learning model, and the learning model data 23 can be updated such that the values of the characteristics obtained by the AI calculation from the input parameter values according to the three-dimensional structure of the porous body become more accurate.

The data conversion unit 18 converts, into three-dimensional shape data 27, the porous body structure data 22 based on the three-dimensional structure of the porous body finally determined by the structure generation unit 11. The three-dimensional shape data 27 generated by conversion from the porous body structure data 22 by the data conversion unit 18 is stored in the storage unit 2, and is read out from the storage unit 2 as necessary and provided to the outside of the porous body design apparatus 100. For example, the three-dimensional shape data 27 is input to a 3D printer, thereby operating the 3D printer in accordance with the three-dimensional shape data 27 to manufacture a porous body that reproduces the three-dimensional structure of the porous body represented by the porous body structure data 22. Thereby, it is possible to create a prototype of the porous body designed by the porous body design apparatus 100. The details of a manufacturing method for a porous body by the 3D printer using the three-dimensional shape data 27 will be described later.

Next, details of processing performed by the control unit 1 will be described with reference to Fig. 2. Fig. 2 is a flowchart showing a flow of processing of the porous body design apparatus according to the embodiment of the present invention. The porous body design apparatus 100 searches for a three-dimensional structure of a porous body having desired characteristics by repeatedly executing the processing shown in the flowchart of Fig. 2 by the control unit 1 in response to a user's operation input. Thereby, the design of a porous body which is performed by the user is supported.

When the user instructs the porous body design apparatus 100 to start to design a porous body via the operation input apparatus 4, the control unit 1 sets an initial value 0 to each of variables i and j in step S10. The variable i is a variable for counting the number of times the AI calculation unit 14 has performed an AI calculation between the time when the physical simulation unit 13 performs a physical simulation and the time when it next performs a physical simulation. The variable j is a variable for counting the number of times the gene evolution unit 16 has performed a gene evolution process, that is, the number of generations of generation parameters that have been set so far by the gene evolution process. While the processing shown in the flowchart of Fig. 2 is being performed, the values of the variables i and j are stored in the memory 3**.**

In step S20, the control unit 1 performs an initial learning model generation process for setting the initial state of the learning model data 23. Here, a large number of samples of the three-dimensional structure of the porous body are acquired using the structure generation unit 11, the model generation unit 12, and the physical simulation unit 13, and a relationship between the values of the input parameters selected from among the structural descriptors of the samples and the characteristics is obtained. Then, the initial state of the learning model data 23 can be set by creating a learning model on the basis of the obtained relationship. Details of the initial learning model generation process will be described later with reference to Fig. 3**.**

In step S30, the control unit 1 sets the initial values of the generation parameters of the porous body by the structure generation unit 11. Here, for example, the values of the generation parameters used when generating the sample of the three-dimensional structure of the porous body in the initial learning model generation process of step S20 are changed randomly within a predetermined range or in accordance with a predetermined rule, thereby setting the initial values of the generation parameters of the porous body. In step S30, the values of the generation parameter that are randomly changed by a preset population number (the number of generation individuals) P are set as the initial values of the generation parameters. For example, when P = 100, 100 combinations of the generation parameter values are set as the initial values of the generation parameters. In addition, the initial values of the generation parameters that are set here are used as a parent generation for generation parameters of a first generation in the gene evolution process of step S150 to be described later. For this reason, in the following description, the initial values of the generation parameters are referred to as generation parameters of a 0-th generation. When the initial values of the generation parameters that are set in this manner are stored in the storage unit 2 as the generation parameter data 21, the processing proceeds to step S40.

In step S40, the control unit 1 causes the structure generation unit 11 to generate a three-dimensional structure of a porous body for which characteristics are to be predicted, on the basis of the initial values of the generation parameters set in step S30 (when j = 0) or generation parameters of a j-th generation which are set in S150 (when j ≥ 1) to be described later. As described above, a three-dimensional structure of a porous body is virtually generated in accordance with the generation parameter values represented by the generation parameter data 21 by using, for example, simulation software such as GeoDict. Thereby, it is possible to generate a three-dimensional structure of a porous body for which characteristics are to be predicted, by the same number as the preset population number P.

In step S50, the control unit 1 causes the structure generation unit 11 to calculate the values of the input parameters corresponding to the three-dimensional structures generated in step S40. Here, as described above, among structural descriptors such as a porosity, a solid fraction, a specific surface area, a pore diameter, a particle diameter, structural uniformity of pores or solid, a pore cord length, and a solid cord length, each structural descriptor that is selected in advance by the structure generation unit 11 as a structural descriptor highly contributing to the characteristics of the porous body is used as an input parameter of the learning model, thereby calculating combinations of input parameter values for the three-dimensional structure of each porous body for which characteristics are to be predicted and which is generated in step S40. When the input parameter values calculated in this manner for each porous body for which characteristics are to be predicted are stored in the storage unit 2 as input parameter data 24, the processing proceeds to step S60.

In step S60, the control unit 1 determines whether the current value of the variable i is a predetermined value M. When i = M, the processing proceeds to step S90, and when i < M, the processing proceeds to step S70. The value of M represents the number of times of setting regarding whether to update the learning model by performing a physical simulation for every several AI calculations, and can be set to any number of 1 or more, for example, M = 10.

In step S70, the control unit 1 causes the AI calculation unit 14 to perform characteristic prediction by AI calculation using the learning model on the basis of the input parameter values calculated in step S50. Here, AI calculation related to characteristic prediction for a porous body is performed by reading out the learning model data 23 to acquire a learning model and inputting, to the learning model, the values of the input parameters obtained from the three-dimensional structure of each porous body for which characteristics are to be predicted. Thereby, a characteristic prediction value is obtained for each combination of input parameters, and characteristic prediction is performed for each porous body for which characteristics are to be predicted. When the characteristic prediction value obtained in this manner for each porous body for which characteristics are to be predicted is stored in the storage unit 2 as characteristic data 25, the processing proceeds to step S80.

In step S80, the control unit 1 adds 1 to the value of the variable i, and causes the processing to proceed to step S120.

In step S90, the control unit 1 causes the physical simulation unit 13 to perform characteristic calculation for each porous body for which characteristics are to be predicted by performing a physical simulation based on the three-dimensional structures generated in step S40 and calculate its characteristic value. When characteristic value calculation results obtained for each porous body for which characteristics are to be predicted are stored in the storage unit 2 as the characteristic data 25, the processing proceeds to step S100.

In step S100, the control unit 1 causes the model update unit 17 to update the learning model represented by the learning model data 23 on the basis of results of the physical simulation performed in step S90. Here, the input parameter data 24 stored in step S50 and the characteristic data 25 stored in step S90 for the porous bodies that have been subjected to the physical simulations performed up to that point are read out from the storage unit 2, and the contents (weight values) of the learning model are updated on the basis of a relationship between the input parameter values and characteristic values for each porous body for which characteristics are to be predicted in this data. When the updated contents of the learning model are recorded in the learning model data 23, the processing proceeds to step S110.

In step S110, the control unit 1 resets the value of the variable i to 0, and causes the processing to proceed to step S120.

In step S120, the control unit 1 causes the characteristic evaluation unit 15 to plot the characteristic values predicted or calculated in step S70 or S90 on a graph for each porous body for which characteristics are to be predicted, and evaluate the characteristic prediction/calculation results. Here, for example, a two-dimensional graph is prepared in which one axis represents a permeability of a fluid, which is one of the characteristics of the porous body, and the other axis represents a filtration efficiency, which is another characteristic of the porous body, and the characteristic values having been predicted or calculated so far for each porous body for which characteristics are to be predicted are plotted on the two-dimensional graph. Thereby, it is possible to evaluate the characteristic prediction/calculation results obtained so far for each porous body for which characteristics are to be predicted, on the basis of the positions of points plotted on the two-dimensional graph. As long as it is possible to evaluate the characteristic prediction/calculation results, it is not necessary to actually plot the points on the graph, and it is only required to store data indicating the positions of the points on the graph. Alternatively, as described above, the characteristic prediction/calculation results may be evaluated using a method other than plotting on the graph.

In step S125, the control unit 1 selects parent generation parameters of the next generation on the basis of the evaluation results of the characteristic prediction/calculation results obtained in step 120. Here, for example, the characteristic values predicted or calculated in the previous and current steps S70 or S90 are relatively evaluated from the positions of the points plotted on the graph in the previous and current steps S120. Then, from these, a predetermined percentage of preferable characteristic values, for example, P characteristic values (P is a population number) equivalent to the top 50%, are selected, and generation parameters of porous bodies corresponding to these characteristic values are selected as parent generation parameters of the next generation, that is, parent generation parameters of a (j + 1)-th generation. However, when the process of step S125 is performed for the first time, that is, when j = 0, there are no previous plot points. For this reason, in this case, all of the initial values of the generation parameters set in step S30 are only required to be selected as parent generation parameters of a 1st generation.

In step S130, the control unit 1 determines whether the current value of the variable j is a predetermined value N. When j = N, and the processing proceeds to step S140. When j < N, the processing proceeds to step S150. The value of N represents the number of times the gene evolution unit 16 executes a gene evolution process, and can be set to any number of 1 or more, for example, N = 100.

In step S140, the control unit 1 determines a final three-dimensional structure of a target porous body on the basis of the evaluation results for the characteristics of the porous body by generation parameters of each generation which have been obtained so far. Here, for example, the characteristic values predicted or calculated in the current step S70 or S90 are relatively evaluated from the positions of the points plotted on the graph in the immediately preceding step S120, and the most highly evaluated characteristic value is specified. A specific method for this will be described later with reference to Fig. 4.

When the most highly evaluated characteristic value can be specified in the process of step S140, the control unit 1 determines a three-dimensional structure of a porous body corresponding to the characteristic value as the final three-dimensional structure of the porous body, and ends the processing shown in the flowchart of Fig. 2.

In step S150, the control unit 1 causes the gene evolution unit 16 to set the next candidate generation parameters. Here, it is possible to set the next candidate generation parameters by performing a gene evolution process based on the generation parameter values selected as the parent generation parameters of the next generation in the immediately preceding step S125, and setting child generation parameters for the parent generation parameters. Specifically, for example, by using a well-known calculation method such as a genetic algorithm, the generation parameters of the j-th generation selected in step S125 are multiplied together to set new generation parameters equivalent to its child as generation parameters of a (j + 1)-th generation. By repeating such operations for a plurality of combinations of parent generation parameters, an arbitrary number of child generation parameters of the next generation are generated. For example, P pairs of parent generation parameters of the j-th generation (P is a population number) are set, and P child generation parameters of the (j + 1)-th generation are created from these pairs as next candidate generation parameters. When the generation parameter values of the (j + 1)-th generation newly obtained by the gene evolution process are recorded in the generation parameter data 21, the processing proceeds to step S160.

In step S160, the control unit 1 adds 1 to the value of the variable j and causes the processing to return to step S40.

After the processing returns to step S40 from step S160, the control unit 1 repeats the process of step S40 and the subsequent processes again. Thereby, a new three-dimensional structure of a porous body for which characteristics are to be predicted is generated on the basis of the generation parameters after the gene evolution process, and characteristic values are predicted or calculated by AI calculation or a physical simulation. Then, by adding plots of the obtained characteristic prediction/calculation results on the graph, new evaluation results for characteristic values of porous bodies based on the generation parameters of the next generation are acquired in addition to the evaluation results for the characteristic values of the porous bodies based on the generation parameters of the previous generation which have been acquired in the processes up to the previous process, and parent generation parameters of the next generation to be used in the gene evolution process are selected from the acquired evaluation results. This series of processes realizes an optimization process for changing generation parameters of a porous body to search for optimal generation parameters.

Fig. 3 is a flowchart showing details of the initial learning model generation process, which is executed in step S20 in Fig. 2.

In step S210, the control unit 1 causes the structure generation unit 11 to set initial generation parameters of the porous body. Here, the initial generation parameters are set in accordance with, for example, a combination of predetermined generation parameter values.

In step S220, the control unit 1 causes the structure generation unit 11 to generate a three-dimensional structure of a porous body used to generate an initial learning model on the basis of the initial generation parameters set in step S210. Here, a three-dimensional structure for generating the initial learning model is generated by the same process as that of step S40 in Fig. 2.

In step S230, the control unit 1 causes the structure generation unit 11 to calculate the values of structural descriptors corresponding to the three-dimensional structure generated in step S220. Here, the values of structural descriptors for the three-dimensional structure of the porous body generated in step S220 are calculated for various predetermined structural descriptors, such as a porosity, a solid fraction, a specific surface area, a pore diameter, a particle diameter, structural uniformity of pores or solid, a pore cord length, and a solid cord length.

In step S240, the control unit 1 causes the physical simulation unit 13 to perform a physical simulation based on the three-dimensional structure generated in step S220. Here, through the same process as step S90 in Fig. 2, characteristics of a porous body having a three-dimensional structure for generating an initial learning model are calculated by a physical simulation, and characteristic values obtained as a simulation result are acquired.

In step S250, the control unit 1 determines whether the processes of steps S210 to S240 have been performed for a predetermined number of samples. When the processes of steps S210 to S240 have been performed for porous bodies corresponding to the predetermined number of samples, and characteristic values for the samples have been calculated, the processing proceeds to step S260. On the other hand, when the number of three-dimensional structures whose characteristic values have been calculated by the processes of steps S210 to S240 is less than the predetermined number of samples and the characteristic values acquired so far do not reach the number of samples, the processing returns to step S210 and perform the process of step S210 and the subsequent processes again.

In step S260, the control unit 1 causes the structure generation unit 11 to select, from among the structural descriptors of the samples calculated in step S230, structural descriptors that are highly correlated with the characteristic values of the porous body obtained by the physical simulation in step S240 as input parameters of the learning model**.** Here, for example, a correlation between the structural descriptor values calculated for each sample and the characteristic values is obtained for each structural descriptor, and a predetermined number of structural descriptors are selected in descending order of obtained correlation values, as input parameters of the learning model**.** Thereby, structural descriptors that contribute highly to the characteristics of a porous body can be selected as input parameters of the learning model**.**

In step S270, the control unit 1 causes the model creation unit 12 to create an initial learning model on the basis of the input parameters selected in step S260 and the characteristic values of the porous body obtained in step **S240.** Here, the initial learning model can be created by performing a model learning process using, for example, a well-known method on the basis of a relationship between the input parameters and characteristic values of each sample.

When the initial learning model is created in step S270, the control unit 1 records the contents of the initial learning model in the learning model data 23 and ends the initial learning model generation process shown in the flowchart of Fig. 3. Thereafter, the processing proceeds to step S30 in Fig. 2.

Fig. 4 is a diagram showing comparison between a three-dimensional structure of a porous body obtained by the porous body design apparatus 100 of this embodiment and a three-dimensional structure of a porous body according to a comparative example. In the graph of Fig. 4, the horizontal axis represents a permeability of a fluid, which is one of the characteristics of a porous body, and the vertical axis represents a filtration efficiency which is another characteristic of the porous body. In the graph of Fig. 4, the value of the permeability on the horizontal axis is normalized such that a minimum value is 0 and a maximum value is 1. In the graph of Fig. 4, each point represented by a point 43 (each point indicated by a • marker, hereinafter referred to as an "example characteristic point") shows an example of a characteristic value for the three-dimensional structure of the porous body according to this embodiment. These example characteristic points are obtained by repeatedly performing calculation and optimization processing of the characteristic values in accordance with the flowchart of Fig. 2. On the other hand, each point represented by a point 41 (each point indicated by a ▪ marker, hereinafter referred to as a "physical calculation characteristic point") shows an example of a characteristic value calculated by creating the same number of three-dimensional structures of the porous bodies as the number of example characteristic points in advance and performing a physical simulation for each three-dimensional structure as a comparative example. In addition, each point represented by a point 42 (each point indicated by a cross marker, hereinafter referred to as an "AI calculation characteristic point") shows an example of a characteristic prediction value obtained only by AI calculation using an initial learning model as another comparative example. These AI calculation characteristic points are obtained by always executing the process of step S70 in the flowchart of Fig. 2 regardless of the value of the variable i.

Comparing the points plotted on the graph of Fig. 4, the AI calculation characteristic points including the points 42 and the example characteristic points including the points 43 are located generally further away from the graph origin in the upper right direction than the physical calculation characteristic points including the points 41. That is, it can be seen that the porous bodies corresponding to these characteristic points have three-dimensional structures with better characteristics than the porous bodies corresponding to the physical calculation characteristic points. As will be described later, when a physical simulation and optimization processing are combined, it is necessary to perform a physical simulation for each of a large number of three-dimensional structures of porous bodies obtained in the process of the optimization processing, which is not realistic because it takes a huge amount of calculation time. On the other hand, the porous body design apparatus 100 of this embodiment can predict characteristic values by using AI calculation in a significantly shorter time than a physical simulation for a large number of three-dimensional structures of porous bodies obtained in the process of optimization processing. For this reason, it is possible to obtain a three-dimensional structure of a porous body having good characteristics such as AI calculation characteristic points and example characteristic points in a realistic calculation time.

The AI calculation characteristic points shown in Fig. 4 represent prediction results of characteristic values obtained when AI calculation is repeatedly performed using the initial state learning model generated in the initial learning model generation process of Fig. 3 as it is. For this reason, there is a high possibility that a deviation from a true characteristic value for the three-dimensional structure of the porous body obtained in the process of the optimization processing will become large, and they do not necessarily represent the three-dimensional structures of the porous bodies having really good characteristics. In addition, due to the deviation from the true characteristic value, there is a possibility that a superiority/inferiority relationship between the characteristic values of the three-dimensional structures cannot be correctly evaluated. On the other hand, in the porous body design apparatus 100 of this embodiment, as described above, a physical simulation is performed every predetermined number of times, and a learning model is updated on the basis of the result. For this reason, the deviation from the true characteristic value for the three-dimensional structure of the porous body obtained in the process of the optimization processing is small. The example characteristic points shown in Fig. 4 are values after updating the physical simulation. Thus, the example characteristic points can reliably obtain three-dimensional structures of porous bodies having good characteristics in terms of a combination of a filtration efficiency and a permeability more reliably than the AI calculation characteristic points.

As described above, in the porous body design apparatus 100 of this embodiment, it is possible to obtain a three-dimensional structure of a porous body having better characteristics than in the comparative example by performing the processing shown in the flowchart of Fig. 2**.**

In the flowchart of Fig. 2, the process of step S120 in which the characteristic values predicted or calculated by AI calculation or a physical simulation are evaluated by a method such as plotting them on a graph, and the gene evolution process of step S150 are repeatedly performed to finally obtain the example characteristic points as shown in Fig. 4. In step S140, a point that shows the best characteristics among these example characteristic points, for example, a point where the normalized values of the permeability and the filtration efficiency are both 0.2 or more and which is located farthest from the graph origin, is selected as the most highly evaluated characteristic value. Then, the final three-dimensional structure of the porous body is determined by specifying the three-dimensional structure corresponding to this characteristic value.

For example, characteristic values of points when a point showing the best characteristics in each of the physical calculation characteristic points, the AI calculation characteristic points, and the example characteristic points is selected from the graph of Fig. 4 are compared with a search time and the number of searched individuals required to search for the points from the physical calculation characteristic points, the AI calculation characteristic points, and the example characteristic points. The comparison results are, for example, as follows. In the following, characteristic values and search times are described when AI calculation characteristic points and example characteristic points are obtained by calculating physical calculation characteristic points for each of 524 types of three-dimensional structures of porous bodies and repeating optimization processing and AI calculation or a physical simulation 10 times until the total number of searched individuals is set to 5000 on the basis of 500 calculation results among them. In this example, a characteristic value and a search time of a point selected from the 524 physical calculation characteristic points are each set to 100, and the characteristic value and the search time of each selected point of the AI calculation characteristic points and the example characteristic points are normalized and shown. Here, the larger the characteristic value, the better the characteristics, and the larger the search time, the longer it takes to perform processing.

### (1) Physical calculation characteristic point

Characteristic value = 100, search time = 100 (total time = 100), number of individuals = 524

### (2) AI calculation characteristic point

Characteristic value = 147, search time per iteration = 6 (total time = 56), number of searched individuals = 5000

### (3) Example characteristic point

Characteristic value = 152, search time per iteration = 15 (total time = 150), number of searched individuals = 5000

From the above comparison results, it can be seen that better characteristics can be obtained in the example characteristic points than physical calculation characteristic points and machine learning characteristic points even in a relatively short search time. The physical calculation characteristic points represent characteristic values calculated by a physical simulation, and thus, similarly to the AI calculation characteristic points and the example characteristic points, when 5000 physical calculation characteristic points are calculated by performing physical simulations on 5000 three-dimensional structures of porous bodies, and a point with good characteristics is selected from among them, there is a possibility that better characteristics than the example characteristic points can be obtained. However, this requires approximately 10 times the search time shown in the example of the physical calculation characteristic points described above, that is, 1000 hours in total time after normalization, which is not realistic. Thus, by adopting a design method for a porous body using the porous body design apparatus 100 of this embodiment, it can be seen that it is possible to obtain characteristic values for a large number of search individuals within a realistic time and to determine a three-dimensional structure of a porous body having desired characteristics within a relatively short time.

Next, details of a method of manufacturing a porous body by a 3D printer using the three-dimensional shape data 27 will be described with reference to Fig. 5**.** Fig. 5 is a flowchart showing a flow of a manufacturing process for a porous body using a 3D printer.

When a user instructs the porous body design apparatus 100 to start to manufacture a porous body via the operation input apparatus 4, the control unit 1 causes the data conversion unit 18 to create three-dimensional shape data 27 in step S310. Here, the porous body structure data 22 is read out from the storage unit 2 and is subjected to a predetermined data conversion process, thereby converting the porous body structure data 22 into the three-dimensional shape data 27. The porous body structure data 22 may be used as the three-dimensional shape data 27 as it is. In this case, the control unit 1 may not include the data conversion unit 18.

In step S320, the three-dimensional shape data 27 created in step S310 is input to a 3D printer (not shown). Here, for example, the porous body design apparatus 100 and the 3D printer are connected in a wireless or wired manner, and the three-dimensional shape data 27 is transmitted from the porous body design apparatus 100 to the 3D printer, thereby inputting the three-dimensional shape data 27 to the 3D printer. Alternatively, the three-dimensional shape data 27 may be input to the 3D printer by moving the three-dimensional shape data 27 from the porous body design apparatus 100 to the 3D printer via a storage medium such as a USB memory. In addition to this, the three-dimensional shape data 27 can be input to the 3D printer by any method.

In step S330, the 3D printer starts to inject a material for forming a porous body on the basis of the three-dimensional shape data 27 input in step S320. Then, in step S340, the head of the 3D printer is moved to the coordinate position represented by the three-dimensional shape data 27 input in step S320, and then the material is injected.

In step S350, it is determined whether a material has been injected from the 3D printer for all three-dimensional structures of the porous body represented by the three-dimensional shape data 27 input in step S320. When there are any three-dimensional structure parts for which the material has not been injected, the processing returns to step S340 to continue injecting the material, and when the material has been injected for all three-dimensional structures, the processing proceeds to step S360.

In step S360, the injection of the material in the 3D printer is ended. Thereby, the manufacture of the porous body using the 3D printer is completed, and a porous body that reproduces the three-dimensional structure designed by the porous body design apparatus 100 can be created. After the process of step S360 is executed, the processing shown in the flowchart of Fig. 5 is ended.

Although an example of a method for manufacturing a porous body by a 3D printer using the three-dimensional shape data 27 has been described above, a porous body that reproduces the three-dimensional structure designed by the porous body design apparatus 100 may be manufactured by other methods. Alternatively, a porous body may be manufactured using something other than a 3D printer. When a porous body that reproduces the three-dimensional structure designed by the porous body design apparatus 100 can be appropriately manufactured using the three-dimensional shape data 27, any method can be selected from among various well-known methods and used to manufacture the porous body.

According to the embodiment of the present invention described above, the following operational effects are achieved.
(1) A method of designing a porous body using the porous body design apparatus 100 causes the porous body design apparatus 100 to execute, a plurality of times, a structure generation process (step S40) of virtually generating a three-dimensional structure of a porous body on the porous body design apparatus 100 on the basis of generation parameter values for generating the porous body, a characteristic prediction/calculation process (steps S70 and S90) of predicting or calculating the characteristics of the porous body having the three-dimensional structure generated by this structure generation process, an evaluation process (step S120) of evaluating the characteristics of the porous body predicted or calculated by this characteristic prediction/calculation process, and an optimization process (steps S125 and S150) of changing the generation parameter values to search for optimal generation parameter values. Then, the three-dimensional structure of the porous body is determined on the basis of the evaluation results of the characteristics of the porous body by the evaluation process (step S140). In this manner, it is possible to provide a useful technology that makes it possible to determine an optimal structure early in the design of a porous body.
(2) In the characteristic prediction/calculation process, at least one of a physical simulation (step S90) and an AI calculation (step S70) is performed. In the physical simulation, the characteristics of the porous body are calculated by physical calculation based on the three-dimensional structure generated by the structure generation process. In the AI calculation, the characteristics of the porous body are predicted using a learning model of the three-dimensional structure. In this manner, it is possible to appropriately perform a physical simulation and AI calculation and determine a three-dimensional structure of the porous body that provides better characteristics.
(3) In the porous body design method, the porous body design apparatus 100 executes an update process (step S100) of updating the learning model on the basis of the results of the physical simulation. In this manner, the learning model can be optimized to obtain more accurate characteristic prediction values compared to a case where AI calculation using the learning model is performed independently.
(4) In the characteristic prediction/calculation process, the physical simulation in step S90 is performed every time the AI calculation in step S70 is performed a predetermined number of times M (step S60: Yes). Then, when the physical simulation in step S90 is performed in the characteristic prediction/calculation process, the porous body design apparatus 100 executes the update process in step S100. In this manner, it is possible to adjust the frequency of updating of the learning model by arbitrarily changing the value of M and determine an optimal structure in a short time with high accuracy.
(5) It is also possible to manufacture a porous body (steps S330 to S360) using three-dimensional shape data based on the three-dimensional structure of the porous body determined by the porous body design method described above. In this manner, it is possible to rapidly and easily create a prototype of the porous body designed by the porous body design apparatus 100.

The present invention is not limited to the above-described embodiment, and can be implemented using any components within the scope of the gist of the invention.

In the above embodiment, any of the structural descriptors representing the three-dimensional structure of the porous body are selected as input parameters of a learning model, and a relationship between the input parameters and the characteristics of the porous body is represented by the learning model. However, instead of the structural descriptors, generation parameters may be used as input parameters of the learning model. In this case, all of a plurality of types of generation parameters for the generation of the porous body may be used as input parameters, or generation parameters highly contributing to the characteristics of the porous body among the plurality of generation parameters may be selected as input parameters. In this manner, even when a process of generating the three-dimensional structure of the porous body and calculating the input parameters (the processes of steps S40 and S50 in Fig. 2) is not performed, the characteristics of the porous body can be directly predicted from the generation parameters by machine learning, which makes it possible to further shorten a processing time.

The above-described embodiments and modification examples are merely examples, and the present invention is not limited to these contents as long as the characteristics of the invention are not impaired. Furthermore, although various embodiments and modification examples have been described above, the present invention is not limited to these contents. Other aspects conceivable within the scope of the technical idea of the present invention are also included in the scope of the present invention.

### [Reference Signs List]

- 1: Control unit
- 2: Storage unit
- 3: Memory
- 4: Operation input apparatus
- 5: Display apparatus
- 6: Bus
- 11: Structure generation unit
- 12: Model creation unit
- 13: Physical simulation unit
- 14: AI calculation unit
- 15: Characteristic evaluation unit
- 16: Gene evolution unit
- 17: Model update unit
- 18: Data conversion unit
- 21: Generation parameter data
- 22: Porous body structure data
- 23: Learning model data
- 24: Input parameter data
- 25: Characteristic data
- 26: Characteristic evaluation data
- 27: Three-dimensional shape data
- 100: Porous body design apparatus

## Claims

1. A porous body design method, which is a design method of porous body using a computer, causing the computer to execute, a plurality of times:
a structure generation process of virtually generating a three-dimensional structure of the porous body on the computer on the basis of generation parameter values for generating the porous body;
a characteristic prediction/calculation process of predicting or calculating characteristics of the porous body having the three-dimensional structure generated by the structure generation process;
an evaluation process of evaluating the characteristics of the porous body predicted or calculated by the characteristic prediction/calculation process; and
an optimization process of changing the generation parameter values to search for optimal generation parameter values, wherein
the three-dimensional structure of the porous body is determined on the basis of evaluation results of the characteristics of the porous body by the evaluation process.

2. The porous body design method according to claim 1,
wherein
in the characteristic prediction/calculation process, at least any of a physical simulation for calculating the characteristics of the porous body by a physical calculation based on the three-dimensional structure generated by the structure generation process, and an AI calculation for predicting the characteristics of the porous body using a learning model of the three-dimensional structure, is performed.

3. The porous body design method according to claim 2,
wherein
the computer is caused to execute an update process of updating the learning model on the basis of results of the physical simulation.

4. The porous body design method according to claim 3,
wherein
in the characteristic prediction/calculation process, the physical simulation is performed every time the AI calculation is performed a predetermined number of times, and
the computer is caused to execute the update process when the physical simulation is performed in the characteristic prediction/calculation process.

5. A porous body manufacturing method of manufacturing the porous body using three-dimensional shape data based on the three-dimensional structure of the porous body determined by the porous body design method according to any one of claims 1 to 4.
